(19) Europäisches Patentamt
European Patent Office
Office européen des brevets

(11) Veröffentlichungsnummer: **0 091 011**
**B1**

(12) # EUROPÄISCHE PATENTSCHRIFT

(45) Veröffentlichungstag der Patentschrift: **05.08.87**

(51) Int. Cl.⁴: **C 07 C 45/71**, C 07 C 47/575, C 07 C 49/84, C 07 C 51/347, C 07 C 65/21

(21) Anmeldenummer: **83102878.2**

(22) Anmeldetag: **23.03.83**

(54) **Verfahren zur Herstellung von Phenyläthern.**

(30) Priorität: **01.04.82 US 364325**

(43) Veröffentlichungstag der Anmeldung: **12.10.83 Patentblatt 83/41**

(45) Bekanntmachung des Hinweises auf die Patenterteilung: **05.08.87 Patentblatt 87/32**

(84) Benannte Vertragsstaaten: **DE FR IT**

(56) Entgegenhaltungen:
**DE - A - 2 211 436**
**FR - A - 2 245 630**
**US - A - 4 065 504**

**Dictionary of organic compounds, 5. Ausgabe (1982), Seiten 1910, 1911, 1915, 2998**

(73) Patentinhaber: **ITT INDUSTRIES INC., 320 Park Avenue, New York, NY 10022 (US)**

(72) Erfinder: **Nelson, Randall Bruce, P.O. Box 393, Shelton Washington 98584 (US)**

(74) Vertreter: **Morstadt, Volker, Dipl.-Ing., c/o Deutsche ITT Industries GmbH Patent/Lizenzabteilung Postfach 840 Hans-Bunte-Strasse 19, D-7800 Freiburg/Brsg. (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Herstellung von Phenyläthern aus den entsprechenden hydroxylgruppenhaltigen Verbindungen.

Es ist hinlänglich bekannt, dass Phenole, insbesondere von Lignin abgeleitete Phenole, unter Verwendung von Alkylsulfaten, Alkylhalogeniden oder Alkylsulfonaten sowohl in protischen wie aprotischen Lösungsmitteln alkyliert werden können. Ein typisches Beispiel einer derartigen Reaktion ist in «Organic Syntheses», Coll. Vol. II, Seite 619, 1943 beschrieben. Darin wird Veratrumaldehyd aus Vanillin, einer aus Lignin stammenden Verbindung, hergestellt. Das bekannte Verfahren bringt die Herstellung von Zwischenstufen aus Natriumsalz oder Phenolat mit einem Alkalihydroxid und Wasser als Lösungsmittel mit sich, woran sich dann die Alkylierungsreaktion des Phenolats mit Methylsulfat zum Phenyläther anschliesst. Dieses Verfahren ist dem in der chemischen Industrie zur Herstellung von Veratrumaldehyd aus Vanillin benutzten ähnlich.

Das bekannte Verfahren ist jedoch teuer und bringt Nachteile mit sich. Das verwendete Alkylierungsmittel muss z.B. ein sehr reaktives Alkylhalogenid oder -sulfat sein. Einige der Alkylierungsmittel erleiden häufig in dem Lösungsmittel eine Hydrolyse und es sind somit Überschussmengen von ihnen erforderlich. Alkylierungsmittel wie z.B. Dimethylsulfat oder Methylchlorid sind äusserst toxisch und erfordern besondere Vorsicht bei ihrer Handhabung.

Ein weiterer Nachteil des bekannten Verfahrens liegt in der Herstellung und Anwendung von Natrium- oder Kalium-phenolat als Zwischenstufe. Die Herstellung und Reaktion dieser Salze erfordert die Anwendung von Lösungsmitteln. Wasser ist billig, seine Verwendung als Lösungsmittel führt jedoch häufig zu parallel laufenden Hydrolysereaktionen, die unvollständige Alkylierungsprodukte ergeben. Vom wirtschaftlichen Standpunkt gesehen verringert die Verwendung eines Lösungsmittels die Menge an Reagenz, entweder Phenol oder Alkylierungsmittel, das in den Reaktor eingegeben wird. Somit leidet der Durchsatz der Reaktion daran, dass erhöhte Arbeitsstunden pro kg Ausbeute erforderlich sind.

Aus der US-A 4 065 504 ist ein Verfahren bekannt, bei dem substituierte Hydroxybenzaldehyde mit Dialkylsulfat in Gegenwart eines Alkalicarbonats umgesetzt werden. Die Reaktion erfolgt unterhalb des Schmelzpunkts der zu alkylierenden Verbindung, wobei das Alkylierungsmittel gleichzeitig als Lösungsmittel fungiert.

Die DE-A 2 211 436 offenbart die Alkylierung von 2-Hydroxy-5-methoxybenzaldehyd mit Dimethyl- oder Diäthylsulfat in Gegenwart von Kaliumcarbonat, wobei Aceton oder das Alylierungsmittel selbst als Lösungsmittel dient.

Hier setzt nun die in den Ansprüchen gekennzeichnete Erfindung ein, deren Aufgabe es ist, ein Alkylierungsverfahren für phenolische Verbindungen anzugeben, das ohne Verwendung eines Lösungsmittels arbeitet und bei erhöhtem Durchsatz der Alkylierungsreaktion ein Endprodukt mit verbesserter Qualität liefert. Die Lösung besteht in einem Verfahren, bei dem zunächst die zu alkylierende phenolische Verbindung, ein mono- oder polyzyklisches Phenol, allein oder zusammen mit dem Katalysator aufgeschmolzen wird und in die Schmelze dann das Alkylierungsmittel sowie der Katalysator, sofern er nicht zuvor schon mitaufgeschmolzen wurde, allmählich zugesetzt wird. Das vorliegende Verfahren ist eine Verbesserung gegenüber den bekannten Verfahren, bei denen die phenolische Komponente mit Alkalihydroxid zur Reaktion gebracht wird und das resultierende Phenolat mit einem Alkylierungsmittel reagieren gelassen wird. Das vorliegende Verfahren ist eine Schmelzphasenreaktion. Die Alkylierung des Phenols wird beim oder oberhalb dessen Schmelzpunkt durchgeführt, wodurch die Notwendigkeit der Herstellung von Alkaliphenolaten und damit die Verwendung eines Lösungsmittels vermieden wird.

Bei der Durchführung des erfindungsgemässen Verfahrens ist die Verwendung einer geringen Menge eines basischen Katalysators notwendig. Die Reaktion läuft zwar ohne einem solchen auch ab, jedoch mit einer sehr verringerten Reaktionsgeschwindigkeit. Man kann jede beliebige, Säuren neutralisierende Base benutzen wie z.B. Alkali oder Erdalkalihydroxide, Amine, Amoniak oder Trinatriumphosphat. Die Alkylierungsreaktion erfordert jedoch weder einen hohen Überschuss noch eine hohe Basizität der verwendeten Base. Man kann einen stark alkalischen Katalysator verwenden, es ist dann weniger als ein Moläquivalent desselben erforderlich. Vorzugsweise verwendet man schwach alkalische Katalysatoren wie z.B. Natrium-, Kalium-, Magnesium- oder Calziumkarbonat. Die für die Reaktion unter Normaldruck erforderliche Menge an Karbonat liegt zwischen 0 und 10 Mol%, bezogen auf die Gesamtmolzahl der umzusetzenden phenolischen Verbindung, obgleich grössere Mengen nicht nachträglich sind. Mengen in der Grössenordnung von 0,1 Mol% sind häufig angebracht. Da kein Lösungsmittel vorhanden ist, kann das Alkylierungsmittel nicht von einem anderen Reagenz aufgebraucht werden und hydrolytische Nebenreaktionen werden vermieden. Dies führt zu einer merklich verbesserten Qualität des Endproduktes.

Die phenolische Ausgangsverbindung ist üblicherweise ein vom Lignin abgeleitetes mono- oder polyzyklisches Phenol. Eine allgemeine Gleichung für die Reaktion ist nachstehend aufgeführt. Sie zeigt die Umwandlung des Phenols in den entsprechenden Äther:

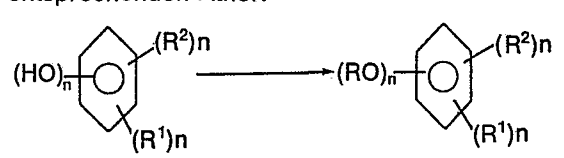

I II

In den Formeln können R1 und R2 gleich oder unterschiedlich und Reste sein wie H, OH, Alkyl,

Alkenyl, Cykloalkyl, Alkoxy, Aryl, Halogen und Karbonyl wie in Aldehyden, Ketonen, Estern, Amiden und Säuren, dabei ist mindestens eines der Reste R1 und R2 vorzugsweise ein –COR3 wobei $R^3$ Wasserstoff, ein Alkyl-, Cykloalkyl- oder Arylrest ist. Der Rest R enthält ein bis vier C-Atome als Alkylgruppe. Der Index n stellt eine Zahl zwischen eins und vier dar. Ist R1 oder R2 ein Arylrest, so kann dieser an ein einzelnes C-Atom des Phenolkerns gebunden sein, (um z.B. Biphenyl zu bilden) oder er kann zwei C-Atome mit einem anderen Kern unter Ausbildung eines polyzyklischen Phenols (z.B. eine Naphtalinverbindung) gemeinsam haben. Die Anordnung der Reste bezüglich der OH-Gruppe und zueinander kann zwischen Ortho-, Meta- oder Parastellung variieren.

Beim alkylierten Endprodukt ist die OH-Gruppe oder sind die OH-Gruppen mit der Alkylhälfte des Alkylierungsmittels unter Ausbildung des entsprechenden Äthers der Ausgangssubstanz alkyliert.

Beispiele für phenolische Verbindungen, die unter die Formel I fallen, sind solche einwertige Phenole wie Phenol selbst und o, m und p-Cresol, phenolische Aldehyde wie Protocatechualdehyd, Vanillin, Synringaldehyd, p-Hydroxy-Benzaldehyd, 5-Formylvanillin und Salicylaldehyd, phenolische Ketone wie p-Hydroxyacetophenon, Acetovanillon, Acetosyringon, Acetamidophenol und Guajacol; und phenolische Säuren wie Vanillinsäure, Syringasäure und p-Hydroxybenzosäure. Die bevorzugten phenolischen Komponenten sind solche mit mindestens einer Karbonylfunktion.

Das Alkylierungsmittel kann im wesentlichen jede Verbindung sein, die als Lieferant einer Alkylgruppe mit ein bis vier C-Atomen dienen kann. Es kann sowohl ein Gas wie eine Flüssigkeit sein. Im allgemeinen sind die Alkylierungsmittel Alkylsulfonate wie Methyl- und Äthyl-p-Toluolsulfonate und Methyl- und Äthylbenzolsulfonate; Trialkylphosphate wie Trimethyl- und Triäthyl-Phosphate; und Dialkylsulfite wie Dimethyl- und Diäthylsulfit. Die Verwendung von Dialkylsulfiten als Alkylierungsmittel in dem vorliegenden Verfahren ist ebenso neu wie die Verwendung von Trialkylphosphaten. Andere Alkylierungsmittel sind Alkylsulfate wie Dimethylsulfat, Diisopropylsulfat und Diäthylsulfat; Alkylhalogenide wie Methylchlorid, Methylbromid, Methyliodid, Äthylchlorid, Äthylbromid, Äthyliodid und ähnliche Propyl- und Butyl-Halogenide.

Beim erfindungsgemässen Verfahren ist es gewöhnlich vorteilhaft, ein Alkylierungsmittel zu wählen, dessen Siedepunkt einige Grade höher liegt als der Schmelzpunkt des zu alkylierenden Phenols, um während der Reaktion auftretende Druckstösse zu steuern. Aus diesem Grund sind Trialkylphosphate besonders vorteilhaft, da ihre Siedepunkte im allgemeinen höher als die Schmelzpunkte der phenolischen Verbindungen liegt.

Das Verfahren nach der Erfindung eignet sich besonders zur Herstellung von methylierten und äthylierten, von Lignin abstammenden phenolischen Verbindungen, die bislang technisch unter Verwendung der sehr gefährlichen Diäthyl- und Dimethylsulfate oder Methyl- und Äthylhalogeniden hergestellt wurden. Das vorliegende Verfahren erlaubt die Verwendung von Chemikalien, die bislang nicht als besonders wirkungsvolle Alkylierungsmittel für Phenole angesehen wurden, wie z.B. Trimethyl-Phosphat und Triäthyl-Phosphat, und die eine bislang in der chemischen Industrie nicht erreichbares Mass an Sicherheit bieten. Das Alkylierungsmittel braucht nicht im Überschuss zugefügt werden, ein geringer molarer Überschuss ist doch vorzuziehen.

In einem bevorzugten Ausführungsbeispiel des Verfahrens nach der Erfindung wird ein vom Lignin abgeleitetes Phenol aufgeschmolzen und mit Alkalikarbonat und einem Alkylierungsmittel in Kontakt gebracht. Letzters liefert die zur Bildung des alkylierten Endproduktes, einem Phenyläther, erforderlichen Alkylreste. Die phenolische Verbindung wird in einer Inertgasatmosphäre unter Zusatz einer kleinen Menge von Alkylikarbonat aufgeschmolzen. Wahlweise wird das Karbonat vor Beginn des Schmelzvorganges zugefügt. Das Alkylierungsmittel wird laufend zugegeben, d.h. fortschreitend mit dem Fortgang der Reaktion, so dass die Reaktionstemperatur nahe der Anfangstemperatur bleibt, das bedeutet ca. 5 bis 10 °C über dem Schmelzpunkt des Phenols, bis ein geringer stöchiometrischer Überschuss von Alkylierungsmittel zugefügt ist (die bevorzugte Menge liegt üblicherweise bei ca. 1,2 Moläquivalente von Alkylierungsmittel bezogen auf Phenol). Nach der Zugabe des Alkylierungsmittels wird die Temperatur eine bestimmte Zeit (mehrere Stunden) zur Sicherstellung einer vollständigen Reaktion konstant gehalten und dann die Mischung auf einen niederen Wert (z.B. 50 °C) abgekühlt und in Wasser eingetragen. Die Gesamtreaktionszeit beträgt normalerweise 1 bis 10 Stunden. Das fertige Produkt kann entweder direkt abgetrennt, oder, falls es ein Öl ist, mit einem geeigneten organischen Lösungsmittel extrahiert und in bekannter Weise rückgewonnen werden. Wahlweise kann das Öl auch von der Reaktionsmischung abgetrennt und nach einer der herkömmlichen Standardmethoden wie z.B. fraktionierte Destillation gereinigt werden. Für viele Zwecke ist jedoch das Rohprodukt hinreichend rein (oftmals 95%), so dass es direkt in dem beabsichtigten Produkt verwendet werden kann.

Die nachfolgenden Beispiele dienen zur Erläuterung der Erfindung. Falls nicht anders angegeben, beziehen sich alle Angaben von Teilen und Prozenten auf das Gewicht.

Beispiel 1

In einer Dreihalsflasche (100 ml), versehen mit einem mechanischen Rührer und Rückflusskühler, wurden 5 g (0,03 mol) Vanillin und 5,0 g (0,036 mol) wasserfreies Kaliumkarbonat unter Stickstoff auf 85 °C erhitzt. Die Mischung war eine klare, bernsteinfarbene Schmelze von Vanillin mit suspendiertem Kaliumkarbonat. Dieser Mischung wurden 5,0 ml (0,043 mol) Trimethylphosphat innerhalb von 5 Minuten zugefügt, wobei die Reaktionstemperatur unter 125 °C gehalten wurde. Die Mischung wurde dann 1 Stunde lang bei 80 °C gehalten und dann auf 40 °C abgekühlt. Das Reaktionsgemisch wurde in 20 ml Wasser eingegossen und zweimal mit 20 ml Methylenchlorid extrahiert. Die vereinigten Extrakte wurden über wasserfreiem Kaliumkarbonat getrocknet, abfiltriert und eingedampft. Es resultierte 5,4 g (99%) Veratrumaldehyd als helles Öl.

Beispiel 2

In einem Gerät wie in Beispiel 1 wurden 15,0 g (0,09 mol) Vanillin und 10,0 g (0,072 mol) wasserfreies Kaliumkarbonat unter Stickstoff auf 90 °C erhitzt. Der klaren Aufschlemmung wurden innerhalb 10 Minuten 13,5 ml (0,112 mol) Trimethyl-Phosphat zugefügt, wobei die Temperatur unter 110 °C gehalten wurde. Der Ansatz wurde unter Rühren drei Stunden auf 65 bis 75 °C erhitzt, dann auf 35 °C abgekühlt und mit 80 ml Wasser zur Abscheidung eines Öles abgeschreckt. Unter Rühren schied sich ein leichtes Pulver ab, das abfiltriert, mit drei mal 100 ml Wasser gewaschen und getrocknet 13,3 g (81%) Veratrumaldehyd ergab.

Beispiel 3

In einem Gerät wie in einer Vorrichtung nach Beispiel 1 wurden 10,0 g (0,045 mol) Syringaldehyd und 5,0 g (0,036 mol) Kaliumkarbonat auf 100 °C erhitzt und 8 ml (0,094 mol) Dimethylsulfit innerhalb von 5 Minuten zugefügt. Die pasteuse Mischung wurde 3 Stunden lang auf 100 °C erhitzt, dann auf 25 °C abgekühlt und mit 50 ml Wasser abgeschreckt. Das Reaktionsgemisch wurde mit 100 ml Methylenchlorid extrahiert, die Extrakte mit Wasser gewaschen und in Vakuum eingedampft. Es resultierte 4,2 g (54% bezogen auf rückgewonnenen Aldehyd) von 3,4,5-Trimethoxybenzaldehyd.

Beispiel 4

In einer Vorrichtung nach Beispiel 1 wurden 15,2 g (0,083 mol) Syringaldehyd und 15,0 g (0,11 mol) Kaliumkarbonat unter Stickstoff auf 105 °C erhitzt und 15 ml (0,12 mol) Trimethylphosphat innerhalb 10 Minuten zugefügt. Das Reaktionsgemisch wurde 3 Stunden lang auf ca. 80 °C gehalten, dann auf 45 °C abgekühlt und mit 50 ml Wasser abgeschreckt. Die ausgefallenen gelbbraunen Feststoffteilchen wurden gesammelt, mit drei mal 50 ml Wasser gewaschen und getrocknet. Es resultierte 15,0 g (92%) 3,4,5-Trimethoxybenzaldehyd.

Es hat sich also gezeigt, dass man einen Alkylierungsprozess erhält, der aufgrund der Durchführung der Reaktion in der Schmelze, es erlaubt, dass der Reaktionsgefässdurchsatz lediglich durch das Volumen der eingesetzten Reaktionsbestandteile begrenzt wird, da kein Verbindungs- oder Lösungsmittel erforderlich ist. Das Fehlen eines Lösungsmittels bedeutet, dass seine Erstehungs-, Rückgewinnungs- und Reinigungskosten vermieden werden. Darüberhinaus ist die Reaktionsfähigkeit der phenolischen Bestandteile in der Schmelzphase, und insbesondere der phenolischen Karbonylverbindungen, derart, dass schwächere Basen und schwächere Alkylierungsmittel grosse Reaktivität zeigen und auf diese Weise ein grösserer Überschuss von Alkylierungsmittel vermieden werden kann.

**Patentansprüche**

1. Verfahren zur Herstellung eines Phenyläthers, wobei ein mono- oder polyzyklisches Phenol in Gegenwart eines basischen Katalysators mit einem Alkylierungsmittel umgesetzt wird, dadurch gekennzeichnet, dass das mono- oder polyzyklische Phenol aufgeschmolzen, danach das Alkylierungsmittel der Schmelze fortlaufend zugeführt und der basische Katalysator entweder dem Phenol vor dem Aufschmelzen oder zusammen mit dem Alkylierungsmittel der Schmelze fortlaufend zugeführt wird.

2. Verfahren nach Anspruch 1, dadurch gekennzeichnet, dass die phenolische Verbindung eine Carbonylgruppe enthält.

3. Verfahren nach Anspruch 2, dadurch gekennzeichnet, dass als phenolische Verbindung ein monozyklisches Hydroxyphenylketon eingesetzt wird.

4. Verfahren nach Anspruch 2, dadurch gekennzeichnet, dass als phenolische Verbindung Vanillin, Syringaldehyd, Salicylaldehyd oder Protocatechualdehyd verwendet wird.

5. Verfahren nach Anspruch 3, dadurch gekennzeichnet, dass als phenolische Verbindung Vanillinsäure, Syringinsäure oder p-Hydroxybenzosäure verwendet wird.

6. Verfahren nach einem der Ansprüche 1 bis 5, gekennzeichnet durch die Verwendung von Methyl- oder Äthylhalogenid als Alkylierungsmittel.

7. Verfahren nach einem der Ansprüche 1 bis 5, dadurch gekennzeichnet, dass als Alkylierungsmittel ein Alkylester mit einer Methyl- oder Äthylgruppe als Alkylrest und eine Sulfat- oder Sulfonatgruppe als Esterrest verwendet wird.

8. Verfahren nach einem der Ansprüche 1 bis 5, gekennzeichnet durch die Verwendung von Trialkylphosphat als Alkylierungsmittel.

9. Verfahren nach einem der Ansprüche 1 bis 5, gekennzeichnet durch die Verwendung von Dialkylsulfit als Alkylierungsmittel.

**Claims**

1. Process for the preparation of a phenolic ether, comprising reacting a mono- or polycyclic phenol in the presence of a basic catalysator with an alkylating agent, characterized in that the mono- or polycyclic phenol is melted, then the alkylating agent is added continuously to the melt and the basic catalysator is added continuously to the phenol either before the melting or together with the alkylating agent to the melt.

2. Process according claim 1, characterized in that the phenolic compound contains a carbonyl group.

3. Process according claim 2, characterized in that the phenolic compound is a hydroxyphenyl keton.

4. Process according claim 2, characterized in that vanillin, syringaldehyde or protocatechualdehyde is used as phenalic compound.

5. Process according claim 3, characterized in that vanillic acid, syringacid or p-hydroxy benzoic acid is used as phenolic compound.

6. Process according to anyone of the claims 1 to 5, characterized by the use of methyl- or ethylhalide as alkylating agent.

7. Process according to anyone of the claims 1 to 5, characterized in that an alkyl ester with a methyl- or ethyl group as an alkyl radical and a sulfate- or sulfonate group as an ester radical is used.

8. Process according to anyone of the claims 1 to 5, characterized by the use of trialkyl phosphate as an alkylating agent.

9. Process according of the claims 1 to 5, characterized by the use of dialkyl sulfite as an alkylating agent.

**Revendications**

1. Procédé de production d'un éther phénylique, dans lequel un phénol mono ou polycyclique est transformé en présence d'un catalyseur basique avec un agent d'alkylation, caractérisé en ce que le phénol mono ou polycyclique est mis en fusion, puis l'agent d'alkylation est incorporé en continu à la fusion et le catalyseur incorporé soit au phénol avant la fusion, soit avec l'agent d'alkylation à la fusion.

2. Procédé conforme à la revendication 1, caractérisé en ce que la liaison phénolique comprend un groupe carbonyle.

3. Procédé conforme à la revendication 2, caractérisé en ce qu'une hydroxyphénylcétone monocyclique est utilisée comme liaison phénolique.

4. Procédé conforme à la revendication 2, caractérisé en ce que de la vanilline, un syringaldéhyde, de l'aldéhyde salicylique ou de l'aldéhyde protocatéchuique sont utilisés comme liaison phénolique.

5. Procédé conforme à la revendication 3, caractérisé en ce que de l'acide vanillique, de l'acide syringique ou de l'acide p-hydroxybenzoïque sont utilisés comme liaison phénolique.

6. Procédé conforme à l'une quelconque des revendications 1 à 5, caractérisé en ce qu'un halogénure de méthyle ou d'éthyle est utilisé comme agent d'alkylation.

7. Procédé conforme à l'une quelconque des revendications 1 à 5, caractérisé en ce qu'un ester d'alkyle avec un groupe méthyle ou éthyle comme résidu d'hydrocarbure saturé est utilisé comme agent d'alkylation et un groupe sulfate ou sulfonate est utilisé comme résidu d'ester.

8. Procédé conforme à l'une quelconque des revendications 1 à 5, caractérisé en ce que du trialkylphosphate est utilisé comme agent d'alkylation.

9. Procédé conforme à l'une quelconque des revendications 1 à 5, caractérisé en ce que du dialkylsulfite est utilisé comme agent d'alkylation.